# EUROPEAN PATENT SPECIFICATION

(11) **EP 1 954 134 B1**
(45) Date of publication and mention of the grant of the patent: **14.09.2011**
(21) Application number: 06827868.8
(22) Date of filing: 17.11.2006
(51) Int. Cl.: A01N 43/42, A61K 31/44

(54) **PROCESS FOR SYNTHESIZING A 2-PHENYL-1H-PHENANTRHO[9,10-D]IMIDAZOLE DERIVATIVE**
VERFAHREN ZUR HERSTELLUNG EINES 2-PHENYL-1H-PHENANTRHO[9,10-D]IMIDAZOL-DERIVATES
PROCEDE DE SYNTHESE D'UN DERIVE 2-PHENYL-1H-PHENANTRHO[9,10-D]IMIDAZOLE

(30) Priority: 23.11.2005 US 739309 P
(43) Date of publication of application: 13.08.2008
(73) Proprietor: Merck Sharp & Dohme Corp., Rahway, NJ 07065 (US)
(72) Inventor: BELYK, Kevin, Michael, Rahway, NJ 07065-0907 (US); DORNER, Benjamin, Rahway, NJ 07065-0907 (US); HARTNER, Frederick, Rahway, NJ 07065-0907 (US); YOSHIKAWA, Naoki, Rahway, NJ 07065-0907 (US); LIMANTO, John, Rahway, NJ 07065-0907 (US); TAN, Lushi, Rahway, NJ 07065-0907 (US); VAZQUEZ, Enrique, Rahway, NJ 07065-0907 (US); PAYACK, Joseph, Rahway, NJ 07065-0907 (US)
(74) Representative: Horgan, James Michael Frederic
(86) International application number: PCT/US2006/044696
(87) International publication number: WO 2007/061853

(56) References cited:
- WO-A1-2005/105814
- WO-A1-2006/063466
- CHO: 'Palladium-catalyzed Sonogashira coupling reaction followed by isomerization and cyclization' JOURNAL OF ORGANOMETALLICS CHEMISTRY vol. 690, no. 17, September 2005, pages 4094 - 4097, XP005027137
- KISHIKAWA ET AL.: 'Highly sensitive and selective determination of 9,10-phenanthrenequinone in airborne particulates using high-performance liquid chromatography with pre-column derivatization and fluorescence detection' JOURNAL OF CHROMATOGRAPHY A vol. 1057, no. 1-2, November 2004, pages 83 - 88, XP005003874

## Description

### BACKGROUND OF THE INVENTION

Modulation of prostaglandin metabolism is at the center of current anti-inflammatory therapies. NSAIDs and COX-2 inhibitors block the activity of cyclooxygenases and their ability to convert arachidonic acid (AA) into prostaglandin (PG) H2. PGH2 can be subsequently metabolized by terminal prostaglandin synthases to the corresponding biologically active PGs, namely, PGI2, thromboxane (Tx) A2, PGD2, PGF2α, and Page2. A combination of pharmacological, genetic, and neutralizing antibody approaches demonstrates the importance of PGE2 in inflammation. In many respects, disruption of PGE2-dependent signalling in animal models of inflammation can be as effective as treatment with NSAIDs or COX-2 inhibitors. The conversion of PGH2 to PGE2 by prostaglandin E syntheses (PGES) may therefore represent a pivotal step in the propagation of inflammatory stimuli.

Microsomal prostaglandin E synthase-1 (mPGES-1) is an inducible PGES after exposure to pro-inflammatory stimuli. mPGES-1 is induced in the periphery and in the CNS by inflammation and represents therefore a novel target for acute and chronic inflammatory disorders. The rational for the development of specific mPGES-1 inhibitors revolves around the hypothesis that the therapeutic utility of NSAIDs and Cox-2 inhibitors would be largely due to inhibition of pro-inflammatory PGE2 while the side effect profile would be largely due to inhibition of other prostaglandin. 2-(Phenyl or heterocyclic)-1h-phenantrho[9,10-d]imidazoles as mPGES-1 inhibitors are disclosed in U.S. No. 60/637,180, filed on December 17, 2004.

WO-A-2005 105814 (Incyte Corporation) discloses the preparation of 2-tert-butyl-9-fluro-3H-benzo[h]imidazo[4,5-f]quinoline by reacting 9-fluorobenzo[h]quinoline-5,6-dione in acetic acid with pivaldehyde and ammonium acetate. The dione is made by adding 9-fluorobenzo[h]quinol-6(5H)- one in THF to a suspension of chromium (VI)oxide absorbed on silica gel.

The present invention describes an efficient and economical process for the preparation of a 2,3-disubstituted 2-phenyl-1h-phenantrho[9,10-d]imidazole derivative that is useful for the large scale production of material for preclinical and clinical use. The current process generates the 2,3-disubstituted 2-phenyl-1h-phenantrho[9,10-d]imidazole derivative in high overall yield.

### SUMMARY OF THE INVENTION

The present invention describes an efficient and economical process for the preparation of a 2,3-disubstituted 2-phenyl-1h-phenantrho[9,10-d]imidazole derivative that is useful for the large scale production of material for preclinical and clinical use. The process of the present invention generates the 2,3-disubstituted 2-phenyl-1h-phenantrho[9,10-d]imidazole derivative in high overall yield. The compound made by the process of the invention is an inhibitor of the microsomal prostaglandin E synthase-I (mPGES-1) enzyme and is therefore useful to treat pain and/or inflammation from a variety of diseases or conditions, such as osteoarthritis, rheumatoid arthritis and acute or chronic pain.

### DETAILED DESCRIPTION OF THE INVENTION

The invention encompasses a process for making a compound of Formula I 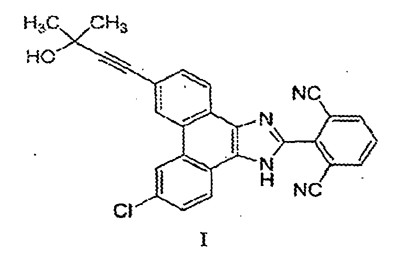 comprising reacting a compound of Formula A 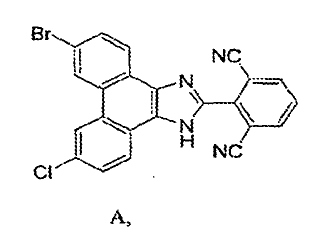 with a compound of Formula B 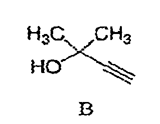 in the presence of a first palladium catalyst which is Pd(OH)₂ on carbon, a copper co-catalyst which is CuI and an amine base which is Et₂N in a suitable solvent to yield a compound of Formula I.

An aspect of the invention encompasses the aforementioned process further comprising making the compound of Formula A by reacting a compound of Formula C 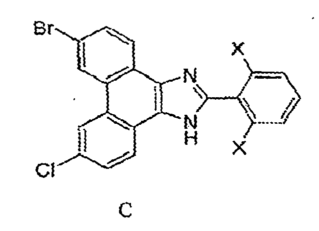 wherein X is a halide with a cyanide salt in a polar solvent to yield a compound of Formula A.

Examples of cyanide salts include NaCN, K₄[Fe(CN)₆], KCN and CuCN. Examples of polar solents include dimethylformamide and 1,3-Dimethyl-3,4,5,6-tetrahydro-2(1H)pyrimidinone.

In an aspect of the invention, X is F or Br, the cyanide salt is selected from the group consisting of: NaCN, K₄[Fe(CN)₆], KCN and CuCN, and the polar solvent is selected from the group consisting of dimethylformamide and 1,3-Dimethyl-3,4,5,6-tetrahydro-2(1H)pyrimidinone.

Another embodiment of the invention encompasses the aforementioned process further comprising masking the compound of Formula C by condensing a compound of Formula D 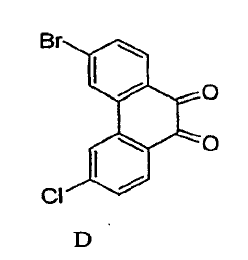 with a compound of Formula E 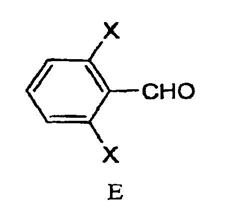 and NH₄OAc in acetic acid to yield a compound of Formula C.

Another embodiment of the invention encompasses the aforementioned process further comprising making the compound of Formula D by reacting a compound of Formula F 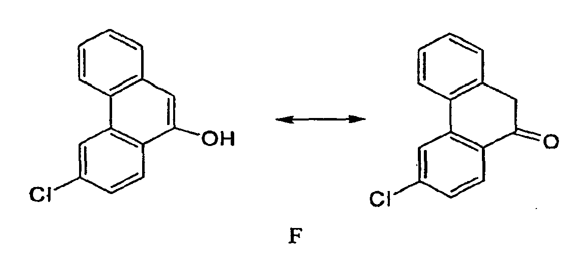 with a brominating agent in a first compatible solvent to make a compound of Formula G 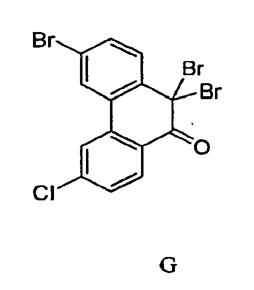 and without further isolation hydrolyzing the compound of Formula G under basic aqueous conditions to make a compound of Formula D.

Brominating agents include for example Br₂ or N-bromosuccinimide. The term "first compatible solvent" means a compatible solvent as discerned by one having ordinary skill in the art. In the instant aspect, "first compatible solvent" includes ethylene glycol dimethyl ether and methyl tertiary-butyl ether.

In an embodiment of the invention, the brominating agent is Br₂ or N-bromosuccinimide and the first compatible solvent is ethylene glycol dimethyl ether or methyl tertiary-butyl ether.

Another embodiment of the invention encompasses the above described process further comprising making the compound of Formula F by cyclizing a compound of Formula H 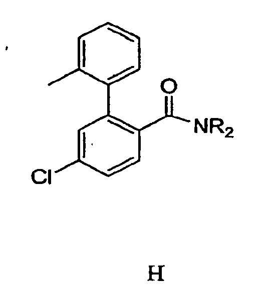 wherein each R is independently C₁-₄alkyl, with a compound of formula MⁱN(Rⁱⁱ)2, wherein Mⁱ is selected from the group consisting of: Li (lithium), Na (sodium) and K (potassium), and each Rⁱⁱ is independently C₁₋₄alkyl, in a second compatible solvent to make a compound of Formula F. The term "second compatible solvent" includes for example ethylene glycol dimethyl ether, tetrahydrofuran and ethers

In an aspect of the invention, the compound of formula MⁱN(Rⁱⁱ)2 is LiNEt₂, and the second compatible solvent is selected from ethylene glycol dimethyl ether, tetrahydrofuran and ethers.

Another embodiment of the invention encompasses the above described process further comprising making a compound of Formula H by coupling a compound of Formula J 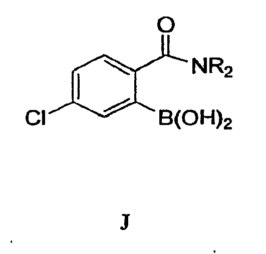 with a compound of Formula K 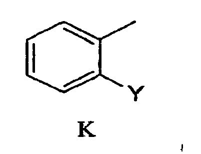 wherein Y is a halide, in the presence of a second palladium catalyst and a base in a third compatible solvent to make a compound of Formula H.

The term "second palladium catalyst" means for example: tetrakis (triphenyl- phosphine) palladium, dichlorobis (triphenylphosphine) palladium and dichloro [1,1'-bis (diphenylphosphine)ferrocene] palladium

The term "base" means for example: Na₂CO₃, K₂CO₃, KHCO₃, NaHCO₃ and an amine.

The term "third compatible solvent" means a compatible solvent as determined by one having ordinary skill in the art, for example: isopropyl alcohol /water, ethylene glycol dimethyl ether /water and toluene/water.

In an embodiment of the invention: the palladium catalyst is selected from the group consisting of: tetrakis (triphenyl- phosphine) palladium, dichlorobis (triphenylphosphine) palladium and dichloro [1,1'-bis (diphenylphosphine)ferrocene] palladium; Y is I; the base is selected from the group consisting of: Na₂CO₃, K₂CO₃, KHCO₃, NaHCO₃ and an amine; and the third compatible solvent is selected from the group consisting of: isopropyl alcohol /water, ethylene glycol dimethyl ether /water and toluene/water.

Another embodiment of the invention encompasses the above described process further comprising making the compound of Formula J, by reacting a compound of Formula L 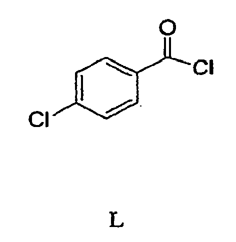 with NHR₂ in an organic non-protic solvent to make a compound of Formula M 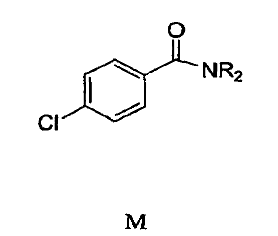 and without further isolation reacting a compound of Formula M with B(O*i*Pr)₃ and MⁱⁱN(Rⁱⁱⁱ)₂, wherein Mⁱⁱ is selected from the group consisting of: Li (lithium), Na (sodium) and K (potassium) and each Rⁱⁱⁱ is independently C₁₋₄alkyl, in a non-reactive solvent to make a compound of Formula J, which can be reacted with the compound of Formula K without further isolation.

The term "organic non-protic solvent" means for example methyl tertiary-butyl ether and the term "non-reactive solvent" means for example ethylene glycol dimethyl ether.

In an embodiment of the invention, MⁱⁱN(Rⁱⁱⁱ)₂ is LíNEt₂, the organic non-protic solvent is methyl tertiary-butyl ether and the non-reactive solvent is ethylene glycol dimethyl ether.

The term "alkyl" means linear or branched structures and combinations thereof, having the indicated number of carbon atoms. Thus, for example, C₁₋₄alkyl includes methyl, ethyl, propyl, 2-propyl, s- and t-butyl, butyl, and 1,1-dimethylethyl.

All references to solvents in the specification include mixtures of solvents.

The compound of Formula I is a selective inhibitor of the microsomal prostaglandin E synthase-1 enzyme and is therefore useful to treat pain and inflammation. Dosage levels range from about 0.01 mg to about 140 mg/kg of body weight per day, including dosage unit forms containing 1, 10 or 100 mg.

The invention will now be illustrated by the following non-limiting example:

### EXAMPLE 1

### Synthetic Scheme to Compound of Formula I

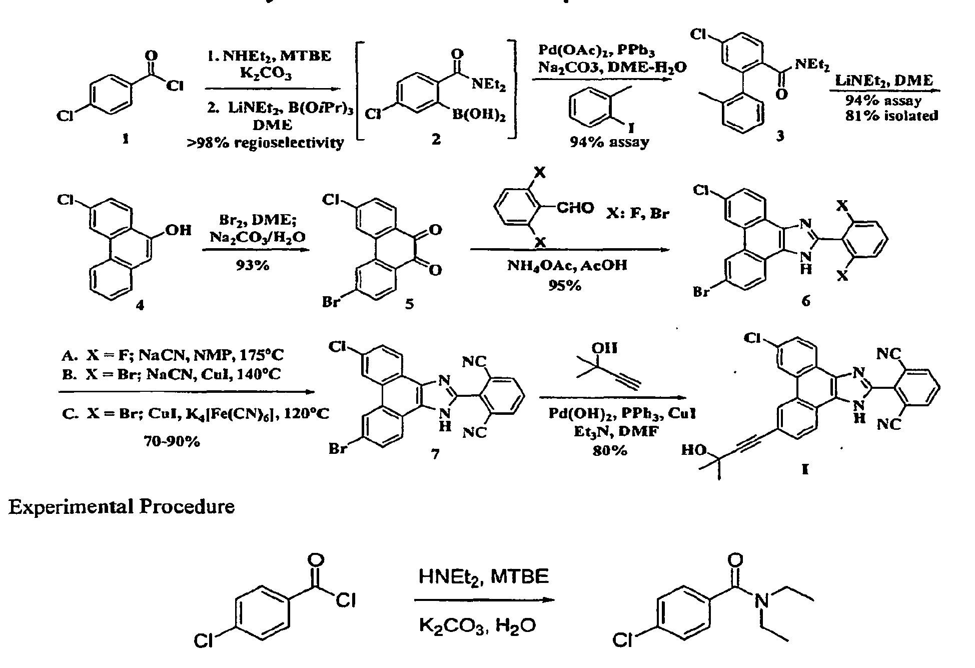

To a round bottom flask was charged potassium carbonate (65g, 469.7 mmol), H₂O (400 mL), MTBE (800) and diethyl amine (81mL, 861.1mmol). *p*-Chlorobenzoyl chloride (100mL, 782.8 mmol) was then added over 30 minutes, maintaining the temperature under 25°C. After addition, the phases were separated and the organics washed with brine (200 mL). The solution was then solvent switched to DME to give a crude solution of the amide, which was used directly in the next step. 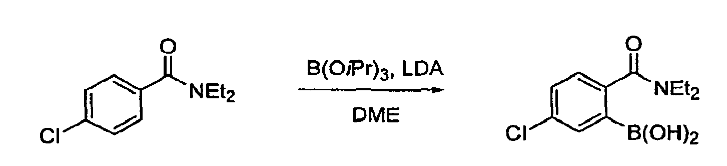 To the crude solution of the amide (10g, 47.3 mmol) in 7.5mL/g DME (75mL) was added triisopropyl borate (19.5 mL, 85.1 mmol) and the resulting solution was cooled to -25°C. A freshly prepared 1.45 M solution of lithium diethylamide (45.6 mL, 66.2 mmol) was then added dropwise over 30 minutes. [NOTE: Lithium diethylamide was generated by treatment of diethylamine in THF with a 2.5M solution of n-butyllithium in hexanes, maintaining the temperature below 0°C during the addition] At the end of addition, the mixture was aged for additional 15 minutes, at which all starting material has been consumed to give the corresponding boronic acid in >98% regioselectivity. The crude solution was then used directly in the next step. 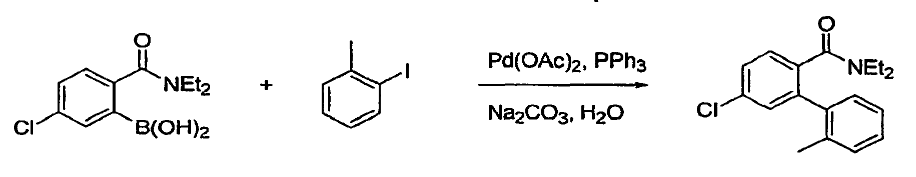 To the crude solution of boronic acid as obtained above was added degassed water (95 mL) at 0°C and solid Na₂CO₃ (13.5g, 127.7 mmol). To the resulting suspension was successively added PPh₃ (223mg, 0.85 mmol), 2-iodotoluene (5.4 mL, 42.6 mmol) and Pd(OAc)₂ (95.5 mg, 0.43 mmol) and the mixture was degassed, heated to 70°C and aged for 6 hours, at which complete consumption of 2-iodotoluene was typically observed. At the end of reaction, MTBE (75mL) was added and the resulting slurry was filtered. Sodium chloride was added to the biphasic filtrate to ease the separation and the layers were cut. The organic phase was washed one time with water (20mL) and brine (2x30mL). The crude solution was then concentrated, solvent switched to DME and used directly in the next step. Typical assay yield: 90-94%. 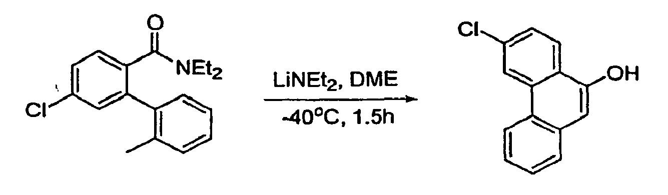 To the crude solution of the amide (13.9g, 46.2 mmol) in 7.5 mL/g DME (104 mL), kept at -45°C, was added freshly prepared 1.44 M solution of LiNEt₂ in THF (41.7 mL, 60 mmol) over 15 min. The resulting brown solution was aged for 75 minutes, at which complete consumption of starting material was observed by HPLC. MTBE (120 mL) was added followed by slow addition of 6N HCl (30.8 mL, 184.7 mmol). The resulting mixture was allowed to warm to RT and the layers were separated (pH of the aqueous layer should be 2-3). The organic layer was washed one time with H₂O (55mL), brine (60mL), concentrated and solvent switched to toluene for crystallization. When approximately 4 mL/g of product in a 3:1 mixture of toluene:DME was obtained, the slurry was refluxed to dissolve all the solid, cooled slowly to 60°C and treated with 5mL/g of methyl cyclohexane (crystals are typically formed at 75-80°C) over 1 hour, while allowing the mixture to cool to RT. The slurry was then concentrated to give a volume of 3.5 mL/g of product and then re-treated with 2 mL/g of methyl cyclohexane over 0.5 hour. The slurry was aged at 0°C for 0.5 hour, filtered and the wetcake was washed with a cold 3:1 mixture of toluene:methyl cyclohexane, followed by drying under constant flow of N₂. The desired product was obtained as light tan solid in 81% yield. 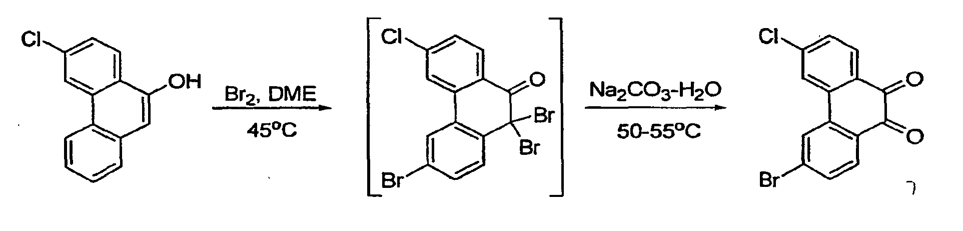 To a solution of chloro-phenanthrole (41g, 179.8 mmol) in dry DME (600mL, KF= 25 ppm, solution KF= 1000 ppm) at 15°C was added Br₂ (32.3 mL, 629.4 mmol) over 20 minutes, at which a 15°C exotherm was evident during the addition. The resulting suspension was then warmed to 40-45°C and aged for 4 hours to give a clear, red solution. A solution of Na₂SO₃ (4.4 g, 36 mmol) in 30 mL of H₂O was added, followed by a solution of Na₂CO₃ (57g, 539.4 mmol) in 250 mL H₂O. The resulting suspension was warmed to 55°C and aged for 5 hour, at which a complete hydrolysis was obtained (additional of H₂O might be necessary to re-dissolve precipitated Na₂CO₃). The reaction mixture was then concentrated at 35-40°C (35-40 torr) to about a third of its volume and the slurry was filtered, washed with H₂O (80-100 mL), followed by 1:1 DME:H₂O (100 mL) and dried under constant flow of N₂. The solid obtained was generally pure enough for the next step; typical yield: 93%. 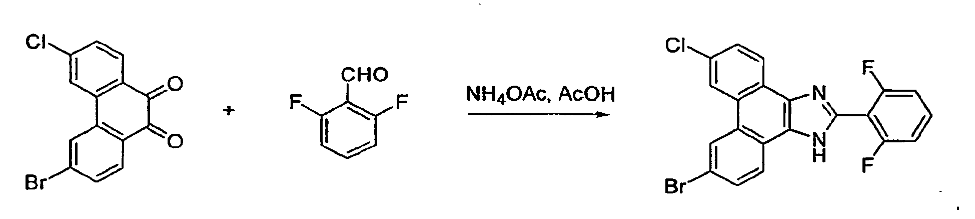 The chlorobromodiketone (4.54g, 14.12 mmol), difluorobenzaldehyde (1.5L, 14.12 mmol), and ammonium acetate (21.77g, 282.38 mmol) were charged to a 250mL round bottom three neck flask under nitrogen. Acetic acid (90mL) was added with stirring, and the slurry was heated to 120°C for 1 hour. The slurry was then cooled to room temperature and water (90mL) was added over 30 min. Upon completion of addition of water, the reaction mixture was filtered, washed with water (45 mL), and dried overnight under nitrogen and vacuum to give the acetic acid salt as a yellow solid.
In order to obtain the freebase, the crude product was dissolved in 1:1 THF/MTBE (90 mL) and charged to a 250mL flask along with 1N NaOH (45 mL). The mixture was then heated to 40°C for one hour. The phases were cut at 40°C, and the organic layer washed with 1N NaOH (45 mL). The organic layer was then concentrated, solvent switched to MTBE, and brought to a final volume of 45mL. The reaction mixture was slurried at 35°C for one hour, cooled to room temperature, filtered, washed with MTBE (23 mL), and dried under nitrogen. The difluoro imidazole freebase (5.97g) was obtained as a light yellow solid in 95% isolated yield. 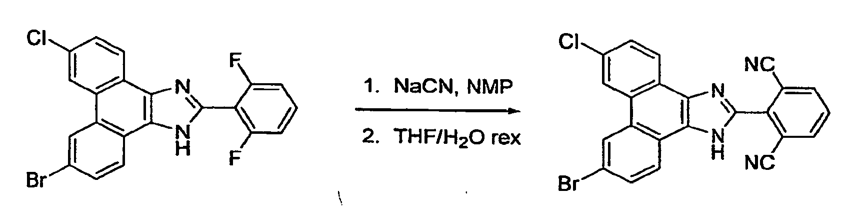 Method A: The difluoroimidazole (6.79g, 13.39 mmol) and sodium cyanide (3.28g, 66.95 mmol) were charged to a 500mL round bottom flask under nitrogen. *N*-methyl pyrrolidone (NMP, 60mL) was added with stirring, and the slurry was heated to 175°C for 28 hours. The reaction mixture was then cooled to room temperature. Water (240mL) was added over 2 hours, and the slurry was allowed to stir for 48 hours. Sodium chloride (36g) was added to the slurry and it was stirred for additional 2 hours. The slurry was then cooled to 0°C, stirred for 1 hour, filtered, and washed with water (30 mL). The wetcake was then dried under nitrogen to give the desired product as NMP solvate.
The solid was slurried in THF (42mL, 7.5mL/g) at 65°C for 1 hour. The mixture was then cooled to room temperature, followed by addition of water (14mL, 2.5 mL/g) over 1 hour. The sl urry was then concentrated under vacuum, removing 14mL of solvent and the resulting slurry was filtered. The wetcake was washed with 1:1 THF/H₂O (14mL), and dried under nitrogen. The desired product (3.83g) was obtained as THF solvate in 54% isolated yield.

### Method B:

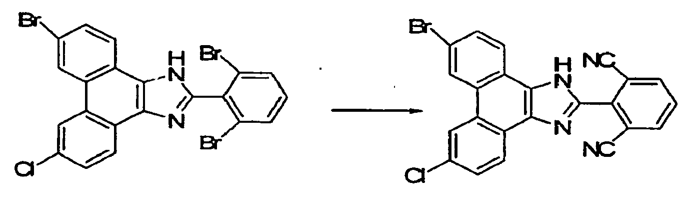

1.0g of tribromoimidazole freebase (1.8 mmol), 260 mg NaCN (5.3 mmol), 135 mg CuI (0.71 mmol) and 7 mL DMF were combined and degassed, then heated to 120°C for 45h. 7 mL of 6:1 water: NH₄OH was added, and the crude product was isolated by filtration. After drying, the material was recrystallized from 1:1 THF:MTBE (16 mL) to afford 870 mg of the dicyano product as the THF solvate (97%).
Method C: tribromoimidazole AcOH salt (1.30 g, 87 wt% as free base, 2 mmol) was treated with K₄[Fe(CN)₆]·3H₂O (845 mg, 2 mmol, finely-powdered), CuI (76.2 mg, 0.4 mmol), and 1,2-phenylenediamine (43.3 mg, 0.4 mmol) in DMF (5.7 mL). The reaction mixture was heated to 135 °C for 36 h, diluted with DMF (5.7 mL), and filtered when hot. The solid was washed thoroughly with acetone, and the washes were combined with the filtrate. The organic solution was concentrated to remove acetone, and H₂O (2.8 mL) was added over 15 min at RT. The resulting solid was collected by filtration, washed with H₂O, and to afford brown solid (1.06 g). The crude solid was then stirred in THF (4 mL) at 60 °C for 1 h and allowed to cool to RT. The resulting solid was collected by filtration, washed with hexane, and dried to afford dicanide THF solvate as off white powder (864 mg, 89.5 wt%).

For Methods B and C above, the tribromoimidazole compound is made following the procedure described above for making the difluoroimidazole compound, but substituting dibromobenzaldehyde for difluorobenzaldehyde.

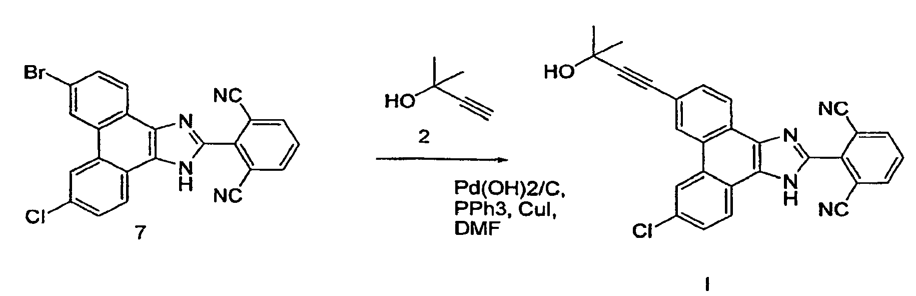

A 7 ml vial, equipped with stir bar and septum screw cap was charged with 6.2 mg of 20wt% Pd(OH)₂ on carbon containing about 16 wt% water (about 1.0 mg Pd(OH)₂ corrected for solid support and water), 69 mg compound 7, 8 mg triphenylphosphine, and 6 mg copper(I) iodide. The vial was brought into a nitrogen filled glovebox where the remaining nitrogen-purged reaction materials were added. NN-Dimethylformamide (0.68 mL) was charged followed by 2-methyl-3-butyn-2-ol (0.022 mL) and triethylamine (0.031 mL). The vial was sealed, removed from the glovebox, placed in a heating block equipped with a nitrogen-purged cover attached, and warmed to an external temperature of 52 °C. The reaction was agitated with heating for about 17 h. HPLC analysis of the reaction at this time showed about 95% LCAP conversion to the compound of formula I using an external reference with >99 LCAP conversion of bromide 7 @ 210nm.

## Claims

1. A process for making a compound of Formula I 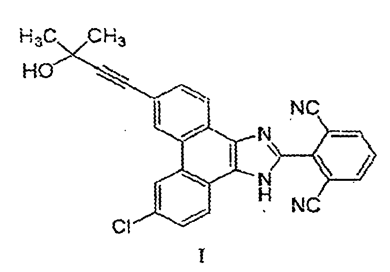 comprising reacting a compound of Formula A 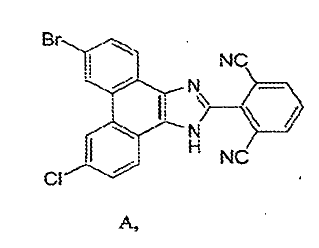 with a compound of Formula B 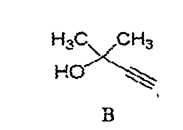 in the presence of a first palladium catalyst which is Pd(OH)₂ on carbon, a copper co-catalyst which is CuI and an amine base which is Et₃N in a suitable solvent to yield a compound of Formula I.

2. The process according to Claim 1 further comprising making the compound of Formula A by reacting a compound of Formula C 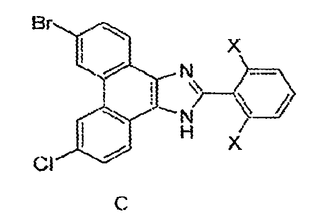 wherein X is a halide with a cyanide salt in a polar solvent to yield a compound of Formula A.

3. The process according to Claim 2, wherein X is F or Br, the cyanide salt is selected from the group consisting of: NaCN, K₄[Fe(CN)₆], KCN and CuCN, and the polar solvent is selected from the group consisting of dimethylformamide and 1,3-Dimethyl-3,4,5,6-tetrahydro-2(1H)pyrimidinone.

4. The process according to Claim 2 or 3 further comprising making the compound of Formula C by condensing a compound of Formula D 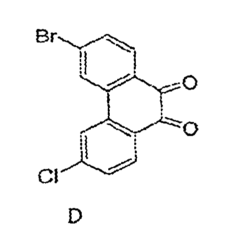 with a compound of Formula E 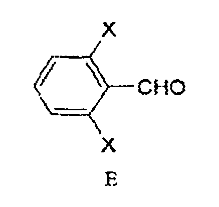 and NH₄OAc in acetic acid to yield a compound of Formula C.

5. The process according to Claim 4, further comprising making the compound of Formula D by reacting a compound of Formula F 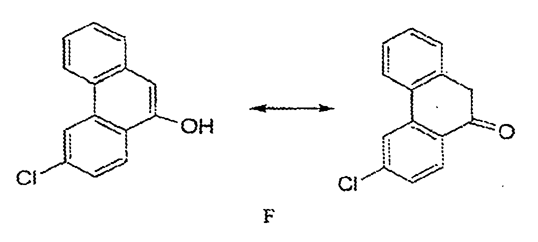 with a brominating agent in a first compatible solvent to make a compound of Formula G 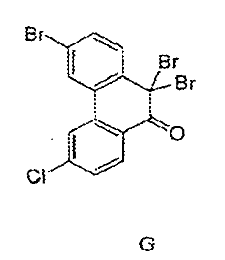 and without further isolation hydrolyzing the compound of Formula G under basic aqueous conditions to make a compound of Formula D.

6. The process according to Claim 5 wherein the brominating agent is Br₂ or N-bromosuccinimide and the first compatible solvent is ethylene glycol dimethyl ether or methyl tertiary-butyl ether.

7. The process according to Claim 5 or 6, further comprising making the compound of Formula F by cyclizing a compound of formula H 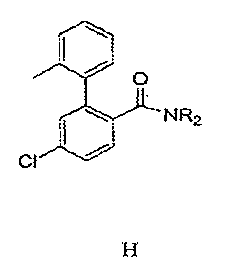 wherein each R is Independently C₁₋₄alkyl, with a compound of formula MⁱN(Rⁱⁱ)2, wherein Mⁱ is selected from the group consisting of: Li, Na and K, and each Rⁱⁱ is independently C₁₋₄alkyl, in a second compatible solvent to make a compound of Formula F.

8. The process according to Claim 7, wherein the compound of formula MⁱN(Rⁱⁱ)2 is LiNEt₂, and the second compatible solvent is selected from ethylene glycol dimethyl ether, tetrahydrofuran and ethers.

9. The process according to Claim 7 or 8, further comprising making a compound of Formula H by coupling a compound of Formula J 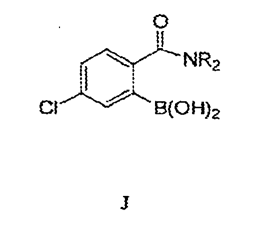 with a compound of Formula K 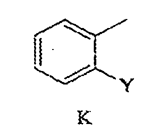 wherein Y is a halide, in the presence of a second palladium catalyst and a base in a third compatible solvent to make a compound of Formula H.

10. The process according to Claim 9, wherein:
the second palladium catalyst is selected from the group consisting of: tetrakis (triphenyl- phosphine) palladium, dichlorobis (triphenylphosphine) palladium and dichloro [1,1'-bis (diphenylphosphine)ferrocene] palladium;
Y is I;
the base is selected from the group consisting of: Na₂CO₃, K₂CO₃, KHCO₃, NaHCO₃ and an amine; and
the third compatible solvent is selected from the group consisting of: isopropyl alcohol/water, ethylene glycol dimethyl ether /water and toluene/water.

11. The process according to Claim 9 or 10, further comprising making the compound of Formula J, by reacting a compound of Formula L 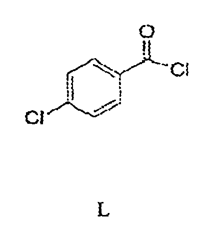 with NHR₂ in an organic non-protic solvent to make a compound of Formula M 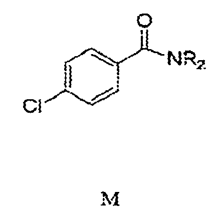 and without further isolation reacting a compound of Formula M with B(O*i*Pr)₃ and MⁱⁱN(Rⁱⁱⁱ)₂, wherein Mⁱⁱ is selected from the group consisting of: Li, Na and K and each Rⁱⁱⁱ is independently C₁₋₄alkyl, in a non-reactive solvent to make a compound of Formula J, which can be reacted with the compound of Formula K without further isolation.

12. The process according to Claim 11 wherein MⁱⁱN(Rⁱⁱⁱ)₂ is LiNEt₂, the organic non-protic solvent is methyl tertiary-butyl ether and the non-reactive solvent is ethylene glycol dimethyl ether.

## Patentansprüche

1. Ein Verfahren zur Herstellung einer Verbindung der Formel I 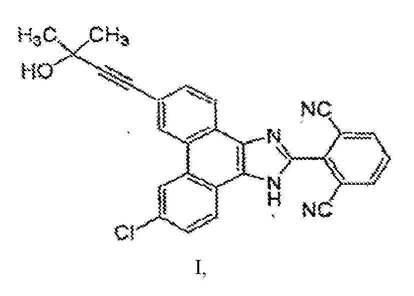 umfassend das Umsetzen einer Verbindung der Formel A 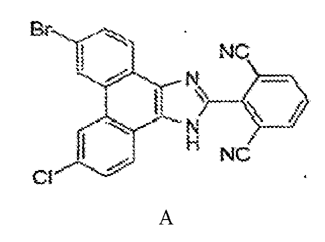 mit einer Verbindung der Formel B 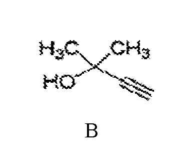 in Gegenwart eines ersten Palladiumkatalysators, der Pd(OH)₂ auf Kohle ist, eines Kupfer-Co-Katalysators, der CuI ist, und einer Aminbase, die Et₃N ist, in einem geeigneten Lösungsmittel, um eine Verbindung der Formel I zu ergeben.

2. Das Verfahren gemäß Anspruch 1, ferner umfassend die Herstellung einer Verbindung der Formel A durch Umsetzen einer Verbindung der Formel C 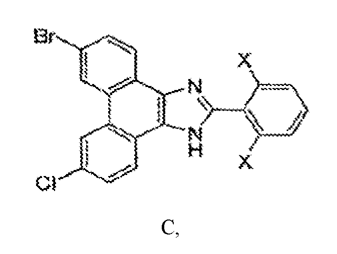 wobei X ein Halogenid ist, mit einem Cyanidsalz in einem polaren Lösungsmittel, um eine Verbindung der Formel A zu ergeben.

3. Das Verfahren gemäß Anspruch 2, wobei X F oder Br ist, das Cyanidsalz ausgewählt ist aus der Gruppe, bestehend aus: NaCN, K₄[Fe(CN)₆], KCN und CuCN, und das polare Lösungsmittel ausgewählt ist aus der Gruppe, bestehend aus Dimethylformamid und 1,3-Dimethyl-3,4,5,6-tetrahydro-2(1H)pyrimidinon.

4. Das Verfahren gemäß Anspruch 2 oder 3, ferner umfassend die Herstellung der Verbindung der Formel C durch Kondensieren einer Verbindung der Formel D 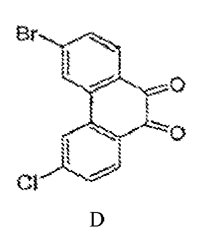 mit einer Verbindung der Formel E 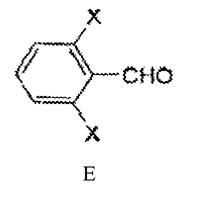 und NH₄OAc in Essigsäure, um eine Verbindung der Formel C zu ergeben.

5. Das Verfahren gemäß Anspruch 4, ferner umfassend die Herstellung der Verbindung der Formel D durch Umsetzen einer Verbindung der Formel F 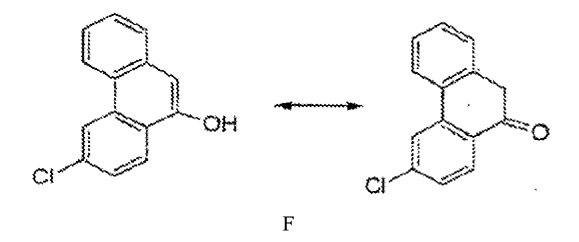 mit einem Bromierungsmittel in einem ersten kompatiblen Lösungsmittel, um eine Verbindung der Formel G zu erzeugen 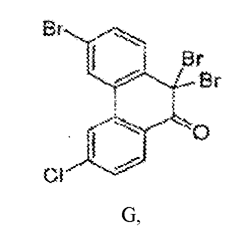 und, ohne weitere Isolierung, Hydrolysieren der Verbindung der Formel G unter basischen wässrigen Bedingungen, um eine Verbindung der Formel D zu erzeugen.

6. Das Verfahren gemäß Anspruch 5, wobei das Bromierungsmittel Br₂ oder N-Bromsuccinimid ist und das erste kompatible Lösungsmittel Ethylenglycoldimethylether oder Methyl-tert-butylether ist.

7. Das Verfahren gemäß Anspruch 5 oder 6, ferner umfassend die Herstellung der Verbindung der Formel F durch Cyclisieren einer Verbindung der Formel H 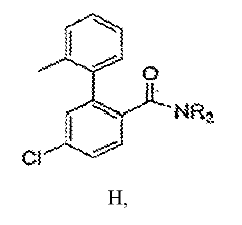 wobei jedes R unabhängig C₁₋₄-Alkyl ist, mit einer Verbindung der Formel MⁱN(Rⁱⁱ)₂, wobei Mⁱ ausgewählt ist aus der Gruppe, bestehend aus: Li, Na und K, und wobei jedes Rⁱⁱ unabhängig C₁₋₄-Alkyl ist, in einem zweiten kompatiblen Lösungsmittel, um eine Verbindung der Formel F zu erzeugen.

8. Das Verfahren gemäß Anspruch 7, wobei die Verbindung der Formel MⁱN(Rⁱⁱ)₂ LiNEt₂ ist und das zweite kompatible Lösungsmittel ausgewählt ist aus Ethylenglycoldimethylether, Tetrahydrofuran und Ethern.

9. Das Verfahren gemäß Anspruch 1 oder 8, ferner umfassend die Herstellung einer Verbindung der Formel H durch Kuppeln einer Verbindung der Formel J 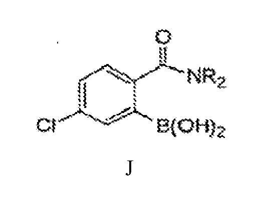 mit einer Verbindung der Formel K 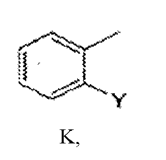 wobei Y ein Halogenid ist, in Gegenwart eines zweiten Palladiumkatalysators und einer Base in einem dritten kompatiblen Lösungsmittel, um eine Verbindung der Formel H zu erzeugen.

10. Das Verfahren gemäß Anspruch 9, wobei:
der zweite Palladiumkatalysator ausgewählt ist aus der Gruppe, bestehend aus:
Tetrakis(triphenylphosphin)palladium, Dichlorbis(triphenylphosphin)palladium und Dichlor[1,1'-bis(diphenylphosphin)ferrocen]palladium,
Y I ist,
die Base ausgewählt ist aus der Gruppe, bestehend aus: Na₂CO₃, K₂CO₃, KHCO₃, NaHCO₃ und einem Amin, und
das dritte kompatible Lösungsmittel ausgewählt ist aus der Gruppe, bestehend aus:
Isopropylalkohol/Wasser, Ethylenglycoldimethylether/Wasser und Toluol/Wasser.

11. Das Verfahren gemäß Anspruch 9 oder 10, ferner umfassend die Herstellung der Verbindung der Formel J durch Umsetzen einer Verbindung der Formel L 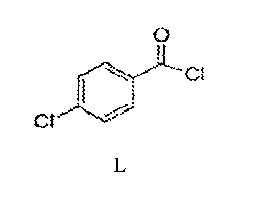 mit NHR₂ in einem organischen nichtprotischen Lösungsmittel, um eine Verbindung der Formel M zu erzeugen 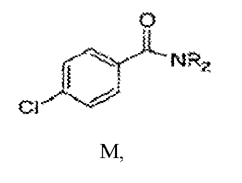 und, ohne weitere Isolierung, Umsetzen einer Verbindung der Formel M mit B(O*i*Pr)₃ und MⁱⁱN(Rⁱⁱⁱ)₂, wobei Mⁱⁱ ausgewählt ist aus der Gruppe, bestehend aus: Li, Na und K, und jedes Rⁱⁱⁱ unabhängig C₁₋₄-Alkyl ist, in einem nichtreaktiven Lösungsmittel, um eine Verbindung der Formel J zu erzeugen, die, ohne weitere Isolierung, mit der Verbindung der Formal K umgesetzt werden kann.

12. Das Verfahren gemäß Anspruch 11, wobei MⁱⁱN(Rⁱⁱⁱ)₂ LiNEt₂ ist, das organische nichtprotische Lösungsmittel Methyl-tert-butylether ist und das nichtreaktive Lösungsmittel Ethylenglycoldimethylether ist.

## Revendications

1. Procédé pour la fabrication d'un composé de la Formule I: 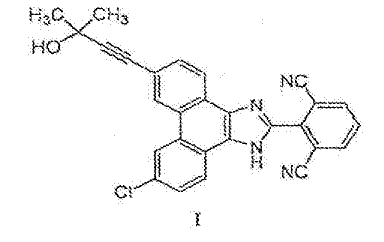 comprenant la réaction d'un composé de la Formule A: 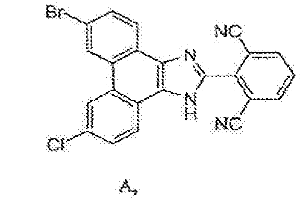 avec un composé de la Formule B: 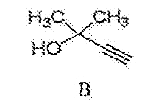 en présence d'un premier catalyseur de palladium qui est Pd(OH)₂ sur un carbone, d'un co-catalyseur de cuivre qui est CuI et d'une base d'amine qui est Et₃N dans un solvant approprié pour donner un composé de la Formule I.

2. Procédé selon la revendication 1, comprenant en outre la fabrication du composé de la Formule A par la réaction d'un composé de la Formule C: 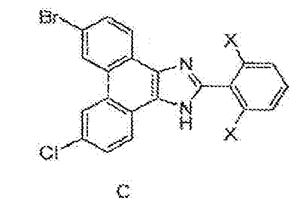 dans laquelle X est un halogénure avec un sel de cyanure dans un solvant polaire pour donner un composé de la Formule A.

3. Procédé selon la revendication 2, dans lequel X est F ou Br, le sel de cyanure est choisi dans le groupe constitué de: NaCN, K₄[Fe(CN)₆], KCN et CuCN, et le solvant polaire est choisi dans le groupe constitué de diméthylformamide et de 1,3-diméthyl-3,4,5,6-tétrahydro-2(1H)pyrimidinone.

4. Procédé selon la revendication 2 ou 3, comprenant en outre la fabrication du composé de la Formule C par la condensation d'un composé de la Formule D: 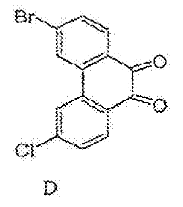 avec un composé de la Formule E: 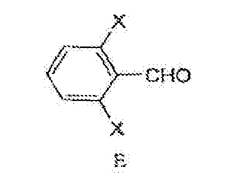 et NH₄OAc dans de l'acide acétique pour donner un composé de la Formule C.

5. Procédé selon la revendication 4, comprenant en outre la fabrication du composé de la Formule D par la réaction d'un composé de la Formule F: 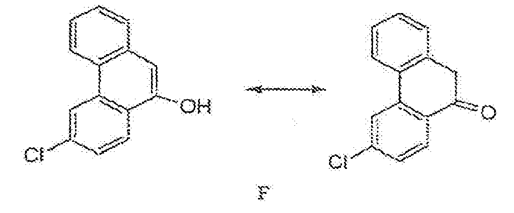 avec un agent de bromation dans un premier solvant compatible pour fabriquer un composé de la Formule G: 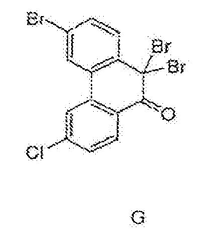 et, sans isolement supplémentaire, l'hydrolyse du composé de la Formule G dans des conditions aqueuses basiques pour fabriquer un composé de la Formule D.

6. Procédé selon la revendication 5, dans lequel l'agent de bromation est Br₂ ou le N-bromosuccinimide et le premier solvant compatible est le diméthyléther d'éthylèneglycol ou l'éther de méthyle de tert-butyle.

7. Procédé selon la revendication 5 ou 6, comprenant en outre la fabrication du composé de la Formule F par la cyclisation d'un composé de la Formule H: 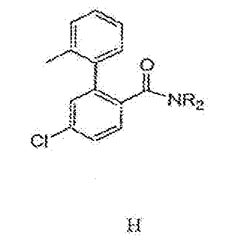 dans laquelle chaque R est indépendamment un alkyle C₁₋₄, avec un composé de la formule MⁱN(Rⁱⁱ)₂, dans laquelle Mⁱ est choisi dans le groupe constitué de: Li, Na et K, et chaque Rⁱⁱ est indépendamment un alkyle C₁₋₄, dans un deuxième solvant compatible pour fabriquer un composé de la Formule F.

8. Procédé selon la revendication 7, dans lequel le composé de la formule MⁱN(Rⁱⁱ)₂ est LiNEt₂ et le deuxième solvant compatible est choisi parmi le diméthyléther d'éthylèneglycol, le tétrahydrofurane et des éthers.

9. Procédé selon la revendication 7 ou 8, comprenant en outre la fabrication d'un composé de la Formule H par le couplage d'un composé de la Formule J: 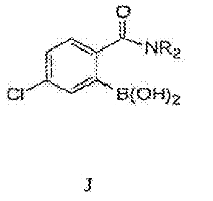 avec un composé de la Formule K: 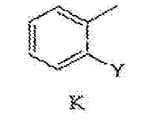 dans laquelle Y est un halogénure, en présence d'un deuxième catalyseur de palladium et d'une base dans un troisième solvant compatible pour fabriquer un composé de la Formule H.

10. Procédé selon la revendication 9, dans lequel:
le deuxième catalyseur de palladium est choisi dans le groupe constitué de: tétrakis(triphényl-phosphine)palladium, dichlorobis(triphénylphosphine)palladium et dichloro[1,1'-bis(diphényl-phosphine)ferrocène]palladium;
Y est I;
la base est choisie dans le groupe constitué de: Na₂CO₃, K₂CO₃, KHCO₃, NaHCO₃ et une amine; et
le troisième solvant compatible est choisi dans le groupe constitué de: alcool d'isopropyle/eau, diméthyléther d'éthylèneglycol/eau et toluène/eau.

11. Procédé selon la revendication 9 ou 10, comprenant en outre la fabrication du composé de la Formule J, par la réaction d'un composé de la Formule L: 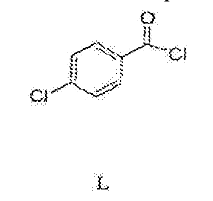 avec NHR₂ dans un solvant organique non protique pour fabriquer un composé de la Formule M: 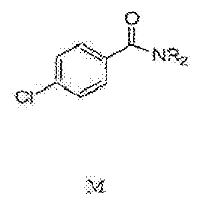 et, sans isolement supplémentaire, la réaction d'un composé de la Formule M avec B(O/Pr)₃ et MⁱⁱN(Rⁱⁱⁱ)₂, où Mⁱⁱ est choisi dans le groupe constitué de: Li, Na et K, et chaque Rⁱⁱⁱ est indépendamment un alkyle C₁₋₄, dans un solvant non réactif pour fabriquer un composé de la Formule J, qui peut être mis à réagir avec le composé de la Formule K sans isolement supplémentaire.

12. Procédé selon la revendication 11, dans lequel MⁱⁱN(Rⁱⁱⁱ)₂ est LiNEt₂, le solvant organique non protique est l'éther de méthyle de tert-butyle et le solvant non réactif est le diméthyléther d'éthylèneglycol.
